# EUROPEAN PATENT APPLICATION

(11) **EP 4 098 296 A1**
(43) Date of publication of application: **07.12.2022**
(21) Application number: 20916449.0
(22) Date of filing: 21.12.2020
(51) Int. Cl.: A61M 5/168

(54) **MEDICAL LIQUID INJECTION DEVICE**

(30) Priority: 31.01.2020 JP 2020015127
(71) Applicant: Asahi Intecc Co., Ltd., Seto-shi, Aichi 489-0071 (JP)
(72) Inventor: NAMIMA, Satoshi, Seto-shi, Aichi 489-0071 (JP)
(74) Representative: Winter, Brandl - Partnerschaft mbB
(86) International application number: PCT/JP2020/047629
(87) International publication number: WO 2021/153091

(57) **Abstract**

A medical liquid injection device includes: a catheter with a hollow shaft having a lumen formed inside to contain medical liquid, a wire to be inserted into the lumen, and a wire moving device to move the wire forward inside the lumen from a proximal end to a distal end side in accordance with a control signal, thereby ejecting a predetermined amount of the medical liquid from the distal end of the hollow shaft.

## Description

### TECHNICAL FIELD

The technology disclosed herein relates to a medical liquid injection device.

### BACKGROUND ART

Several devices have been known which inject medical liquid into the body of a patient using a catheter capable of being inserted into the living body lumen. Patent Literature 1, for example, discloses an injection device including an injection catheter and a hand pump connected to the proximal end of the injection catheter. Patent Literature 2, for example, discloses an infusion pump including a catheter communicating with an infusion tube, a driving motor, and a liquid delivery driver part that presses the infusion tube to send medical liquid in the infusion tube to the catheter by positively rotating a rotor of the driving motor.

### CITATION LIST

### Patent Literature

Patent Literature 1: JP 2001-519212 A
Patent Literature 2: WO2014/049661

### SUMMARY OF INVENTION

### TECHNICAL PROBLEM

Various doses are employed for medical liquid to be injected into the body of a patient. In this regard, the technologies described in Patent Literatures 1 and 2 have a problem of lacking precise control of an injection amount of medical liquid, thus failing to meet request for injection of a small amount of medical liquid into the body of a patient. The technologies described in Patent Literatures 1 and 2 also have a problem in that medical liquid remains inside an injection catheter, an infusion tube, and the like after use, thus leading to waste of the medical liquid. In addition, the technology described in Patent Literature 2 makes a medical liquid injection device more extensive, thus preventing easy installation and settings.

An object herein is to provide an automated medical liquid injection device for solving at least a part of the problems mentioned above as well as achieving reduced burden and promoted efficiency of a procedure of a practitioner.

### SOLUTION TO PROBLEM

The technology disclosed herein can be implemented, for example, as the following aspect.
(1) A medical liquid injection device disclosed herein is a medical liquid injection device including: a catheter with a hollow shaft having a lumen formed inside to contain medical liquid, a wire to be inserted into the lumen, and a wire moving device to move the wire forward inside the lumen from a proximal end to a distal end side in accordance with a control signal, thereby ejecting a predetermined amount of the medical liquid from the distal end of the hollow shaft.
   The medical liquid injection device includes a wire to be inserted into a lumen to contain medical liquid, wherein the wire is moved forward from the proximal end to the distal end side of the lumen, thereby ejecting the medical liquid in the lumen from the distal end of the hollow shaft. That is, the configuration pushes out the medical liquid in the lumen by the wire, thereby achieving ejection of the medical liquid from the distal end of the hollow shaft. This allows an amount of the medical liquid to be ejected to have the same amount as the volume of the wire, thus providing precise control of an injection amount of the medical liquid. The configuration can also push out the medical liquid in the lumen by the wire, thereby allowing full use of the medical liquid in the lumen. This enables prevention of waste of the medical liquid. The medical liquid injection device also includes a wire moving device that moves the wire forward into the lumen in accordance with a control signal. This allows the medical liquid injection device to automatically execute forward movement of the wire into the lumen. Accordingly, the medical liquid injection device enables automatic injection of medical liquid with precisely controlling an injection amount of the medical liquid to reduce waste of the medical liquid.
(2) The medical liquid injection device described above may be configured such that the catheter has a hollow needle part at the distal end of the hollow shaft. In the medical liquid injection device having such configuration, puncture can be made with a needle part at a position of interest inside the body of a patient (e.g., affected area) when medical liquid is injected at the position of interest. Therefore, the medical liquid injection device enables fixation of the needle part to a position of interest inside the body of a patient, thus allowing precise injection of medical liquid at the position of interest, as well as injection of the medical liquid inside the position of interest.
(3)The medical liquid injection device described above may be configured such that the wire moving device has a wire driver part to move the wire forward, a distance detection sensor to detect an advance distance of the wire, and a control device, wherein the control device terminates operation of the wire driver part upon determining that the advance distance of the wire derived based on a detection result of the distance detection sensor is a distance corresponding to the predetermined amount. In terminating operation of the wire driver part, the medical liquid injection device including such configuration enables more precise termination of operation of the wire driver part by using an advance distance of the wire derived based on a detection result of a distance detection sensor.
(4) The medical liquid injection device described above may be configured such that the wire moving device has the wire driver part removably configured with the control device. The medical liquid injection device including such configuration allows automatic forward movement of the wire into the lumen in a configuration with the control device attached to the wire driver part, as well as manual forward movement of the wire into the lumen in a configuration with the control device removed from the wire driver part. For example, in injection of medical liquid on the surface or inside (hereinafter referred to as "the surface or the like") of an affected area in the body of a patient, a practitioner can manually move the wire forward to the lumen, thereby obtaining a sense of the surface or the like of the affected area. The practitioner can recognize, based on this sense, whether the surface or the like of the affected area is suited for injection of medical liquid. For example, injection pressure of medical liquid in manually moving the wire forward to the lumen provides a sense of hardness of the surface or the like of the affected area, and then this sense can provide a basis to move a position for injecting medical liquid to another position in the affected area when the affected area has a certain hardness on the surface or the like (has hardness unsuitable for injecting medical liquid).
(5) The medical liquid injection device described above may be configured such that the wire driver part moves the wire forward by sending the wire to the lumen and moves the wire backward by winding up the wire from the lumen. The medical liquid injection device including such configuration allows the wire driver part to move the wire forward by sending the wire to the lumen and to move the wire backward by winding up the wire from the lumen, thus ensuring forward and backward movement of the wire. In addition, since the wire is wound up to be stored, a space required for storing the wire (a storing part, described later) can be miniaturized.
(6) The medical liquid injection device described above may be configured such that the wire driver part has a wire winding part having winding of a portion of the proximal end side of the wire, and a handle part connected to the wire winding part, wherein the wire winding part sends and winds up the wire in a length corresponding to a rotation amount of the handle part. The medical liquid injection device including such configuration allows sending and winding-up of a wire by rotating a handle part, thus providing the medical liquid injection device with a simple configuration.
(7) The medical liquid injection device described above may be configured to include a storing part connected to the proximal end part of the catheter and containing a portion of the proximal end side of the wire and a portion of the wire driver part. The medical liquid injection device including such configuration includes a storing part connected to the proximal end part of the catheter, storing a portion of the proximal end side of the wire, as well as storing a portion of the wire driver part that enables forward and backward movement of the wire inside the lumen. This allows a practitioner to move the wire forward and backward without directly touching the wire.
(8) The medical liquid injection device described above may be configured such that the wire moving device further includes a wire extrusion part to extrude toward the lumen the wire sent from the wire winding part, wherein the wire extrusion part includes a first roller and a second roller to rotate corresponding to rotation of the handle part, and holds and extrudes the wire by the first roller and the second roller. In the medical liquid injection device including such configuration, the wire moving device includes the wire extrusion part to extrude toward the lumen the wire sent from the wire winding part, thus allowing the wire to be securely pushed forward into the lumen.
(9) The medical liquid injection device described above may be configured such that the storing part further includes a valve member, wherein the valve member is disposed between the wire extrusion part and the proximal end part of the catheter and regulates migration of medical liquid in the lumen into the storing part. The medical liquid injection device including such configuration enables preventing medical liquid in a lumen from entering the storing part, and allows maintaining airtightness in the storing part.
(10) The medical liquid injection device described above may be configured such that the catheter further includes a connector, wherein the connecter has a medical liquid supply port for supplying medical liquid to the lumen and a wire insertion opening for inserting the wire into the lumen, wherein the medical liquid supply port is connected to the proximal end part of the hollow shaft and communicates with the lumen, and wherein the medical liquid supply port is disposed closer to the distal end than the distal end of the wire in maximal winding-up of the wire to the wire winding part by rotation of the handle part. In the medical liquid injection device including such configuration, the medical liquid supply port of the connector is disposed closer to the distal end than the distal end of the wire in maximal winding-up of the wire to the wire winding part. This allows supply of medical liquid from the medical liquid supply port without inhibition by the wire, when the handle part is rotated to maximally wind up the wire to the wire winding part.
(11) The medical liquid injection device described above may be configured such that the connector further includes a first extension part extending in a longitudinal direction of the catheter, and a second extension part extending from a lateral face of the first extension part in the vicinity of the distal end of the first extension part in a direction different from that of the first extension part, wherein the medical liquid supply port is formed in a distal end part of the second extension part, and wherein the wire insertion opening is formed in a proximal end part of the first extension part. In the medical liquid injection device including such configuration, the connector includes the wire insertion opening formed in the proximal end part of the first extension part, and the medical liquid supply port formed in the distal end part of the second extension part extending from a lateral face of the first extension part in the vicinity of the distal end of the first extension part. This enables smooth insertion of the wire into the wire insertion opening and supply of medical liquid from the medical liquid supply port.
(12) The medical liquid injection device described above may be configured such that the outer diameter of the wire is approximately constant from the distal end to the proximal end. The medical liquid injection device including such configuration allows easy recognition of an amount of medical liquid to be ejected from the distal end of the hollow shaft (or the needle part) corresponding to a length of the wire to be moved inside the lumen.
(13) The medical liquid injection device described above may be configured such that each of a cross-sectional shape of the lumen and a cross-sectional shape of the wire is approximately circular, and that the outer diameter of the wire is approximately the same as the inner diameter of the lumen. The medical liquid injection device including such configuration enables the wire to push out medical liquid in the lumen with maintaining slidability of the wire inside the lumen.

The technology disclosed herein can be implemented in a variety of aspects, for example, in an aspect such as a medical liquid injection device, a catheter and a wire for medical liquid injection, a wire and a wire moving device for medical liquid injection, a system including a medical liquid injection device, a system including a wire and a wire moving device, or a method of producing these devices and systems thereof.

### BRIEF DESCRIPTION OF DRAWINGS

Fig. 1 is an explanatory diagram illustrating a configuration of a medical liquid injection device 1 in a first embodiment.
Fig. 2 is an explanatory diagram depicting a cross-sectional configuration of a catheter 10.
Fig. 3 is an explanatory diagram illustrating a configuration of a wire 80.
Fig. 4 is an explanatory diagram illustrating a configuration of the wire 80.
Fig. 5 is an explanatory diagram illustrating a configuration of a wire moving device 50 and a storing part 60.
Fig. 6 is an explanatory diagram illustrating a configuration of a wire winding part 511, a gear 513, and a wire extrusion part 514.
Fig. 7 is an explanatory diagram illustrating a configuration of a valve member 62.
Fig. 8 is an explanatory diagram illustrating a configuration of a CPU built-in case 53.
Fig. 9 depicts an example of a configuration of a driving control device 533.
Fig. 10 is a flowchart depicting an example of a processing flow of the driving control device 533.
Fig. 11 is a flowchart depicting an example of a processing flow of step S21.
Fig. 12 illustrates forward movement of the wire 80 and ejection of medical liquid.
Fig. 13 illustrates backward movement of the wire 80.
Fig. 14 is an explanatory diagram illustrating a configuration of a medical liquid injection device 1A in a second embodiment.
Fig. 15 is an explanatory diagram illustrating a configuration of a wire 80A.
Fig. 16 is an explanatory diagram illustrating a configuration of a medical liquid injection device 1B in a third embodiment.
Fig. 17 is an explanatory diagram illustrating a configuration of a CPU built-in case 53B.

### EMBODIMENTS OF THE INVENTION

### A. First Embodiment:

### A-1. Configuration of Medical Liquid Injection Device 1:

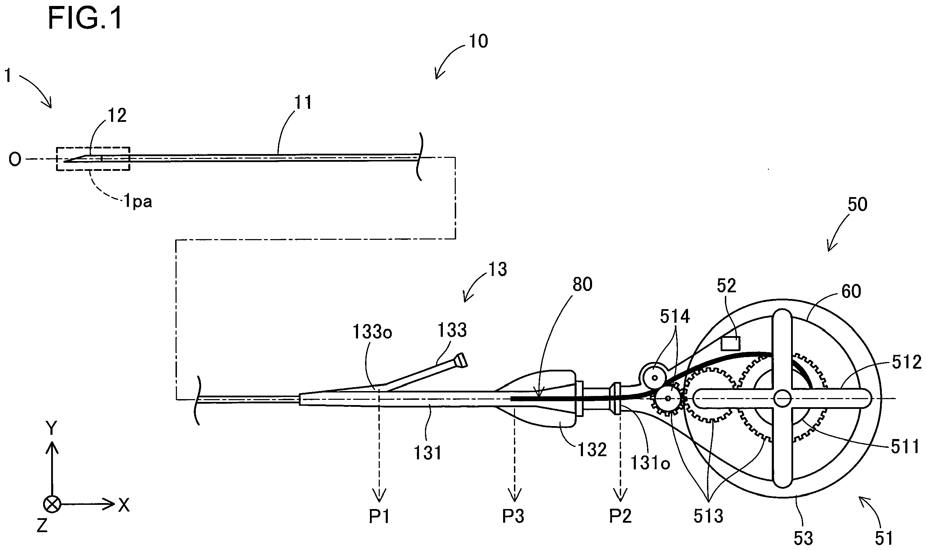

Fig. 1 is an explanatory diagram illustrating a configuration of a medical liquid injection device 1 in a first embodiment. The medical liquid injection device 1 is a device used for injecting medical liquid into the body of a patient using a catheter capable of being inserted into the living body lumen. Note that the living body lumen includes a variety of lumens such as the blood vessel system, lymph gland system, biliary tract system, urinary tract system, respiratory tract system, digestive organ system, secretory gland, and genital organ. The medical liquid injection device 1 includes a catheter 10, a wire moving device 50, a storing part 60, and a wire 80.

Fig. 1 shows an axis passing through the center of the catheter 10 (more particularly, a needle part 12 and a hollow shaft 11, described later) in the medical liquid injection device 1 by an axis line O (dash-dotted line). In the example in Fig. 1, the axis line O is identical with an axis passing across each center of a wire winding part 511, a handle part 512, and a gear 513, which configures the wire moving device 50 and is described later. However, the axis line O may be different from each central axis of the catheter 10 and the wire moving device 50. Fig. 1 depicts X-, Y-, and Z-axes perpendicular to one another. The X-axis corresponds to the length direction of the medical liquid injection device 1, the Y-axis corresponds to the height direction of the medical liquid injection device 1, and the Z-axis corresponds to the width direction of the medical liquid injection device 1. The left side in Fig. 1 (-X-axis direction) refers to as "distal end side" of the medical liquid injection device 1 and each configuration member, and the right side in Fig. 1 (+X-axis direction) refers to as "proximal end side" of the medical liquid injection device 1 and each configuration member. In the medical liquid injection device 1 and each configuration member, the end part located on the distal end side refers to as "distal end", and the distal end and the vicinity thereof refer to as "distal end part". Meanwhile, the end part located on the proximal end side refers to as "proximal end", and the proximal end and the vicinity thereof refer to as "proximal end part". The distal end side is inserted into the living body, and the proximal end side is manipulated by a practitioner such as a doctor. These points are also common subsequent to Fig. 1.

### A-2. Configuration of Catheter 10:

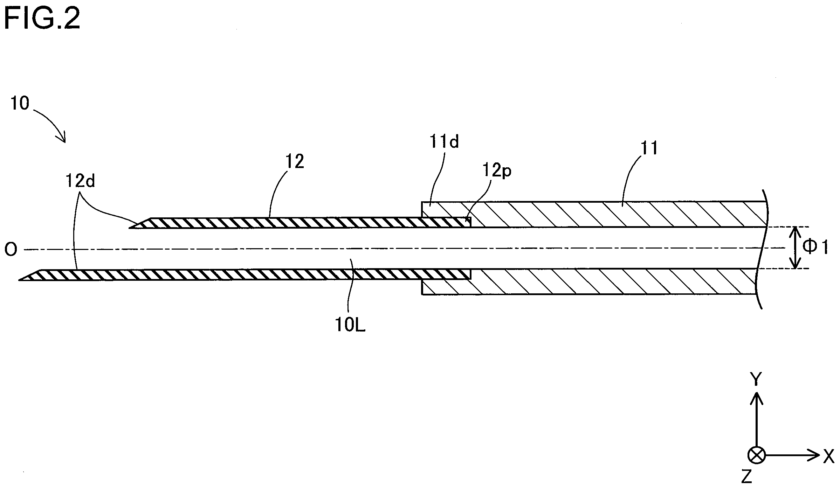

Fig. 2 is an explanatory diagram depicting a cross-sectional configuration of the catheter 10. Fig. 2 shows a cross-sectional configuration of a portion 1pa on the distal end side of the catheter 10 (Fig. 1: dashed-line box). The X-, Y-, and Z-axes in Fig. 2 correspond to the X-, Y-, and Z-axes in Fig. 1. The catheter 10 is inserted into the living body lumen of a patient, and used for conveying medical liquid to a desired position in the body of the patient and injecting the medical liquid into the desired position in the body of the patient. The catheter 10 includes the hollow shaft 11, the needle part 12, and a connector 13.

The hollow shaft 11 is a long-shaped member extending along the axis line O. The hollow shaft 11 has a lumen 10L formed inside to contain medical liquid. More particularly, the hollow shaft 11 takes a hollow, approximately cylindrical shape where an opening is formed on each of the distal end part and the proximal end part, and where an inner lumen (lumen 10L) interconnecting both of the openings is formed inside. In addition, a coil body, a braided body, or the like may be embedded in a thickness part of the hollow shaft 11, for improving at least partially flexibility, torquability, pushability, kind resistance, blood vessel trackability, lesion crossability, and operability of a guide wire. The hollow shaft 11 may also be configured of a plurality of layers formed of the same material or different materials. A cross-sectional shape of the lumen 10L is approximately circular.

The needle part 12 is a hollow needle in which an opening is formed in each of a distal end part 12d and a proximal end part 12p, and inside which an inner lumen interconnecting both of the openings is formed (see Fig. 2). The inner lumen of the needle part 12 is integrated with the inner lumen of the hollow shaft 11 to configure the lumen 10L. In the needle part 12, the distal end part 12d has a sharp needle tip formed thereon, and the proximal end part 12p is jointed to a distal end part 11d of the hollow shaft 11. The joining of the needle part 12 can be made with an any bond such as an epoxy-based adhesive. The needle part 12 is jointed to the hollow shaft 11 with interconnecting the inner lumen of the needle part 12 and the inner lumen of the hollow shaft 11, and the inner lumen of the needle part 12 and the inner lumen of the hollow shaft 11 both have an approximately constant inner diameter Φ1.

The connector 13 is connected to the proximal end part of the hollow shaft 11, and used for supply of medical liquid to the catheter 10 (more particularly, the hollow shaft 11 and the needle part 12) and insertion of the wire 80 to the catheter 10 (more particularly, the hollow shaft 11 and the needle part 12). The connector 13 includes a first extension part 131, a blade part 132, and a second extension part 133.

The first extension part 131 is an approximately hollow-cylindrical member connected to the proximal end part of the hollow shaft 11 and extending along the axis line O (i.e., in the longitudinal direction of the catheter 10). Inside the first extension part 131, an inner lumen is formed that extends along the axis line O. In the example in Fig. 1, not only the inner lumen of the needle part 12 and the inner lumen of the hollow shaft 11, which are described above, but also the inner lumen of the first extension part 131 configures the lumen 10L. In the proximal end part of the first extension part 131, a wire insertion opening 131o is formed that interconnects the lumen 10L to outside. The second extension part 133 is an approximately hollow-cylindrical member extending from a lateral face of the first extension part 131 in a direction different from that of the first extension part 131 in the vicinity of the distal end of the first extension part 131. Inside the second extension part 133, an inner lumen is formed that communicates with the lumen 10L. In the distal end part of the second extension part 133, a medical liquid supply port 133o is formed that interconnects the lumen 10L and the inner lumen of the second extension part 133.

As shown in Fig. 1, the catheter 10 in the embodiment includes a position P1 of the medical liquid supply port 133o disposed closer to the distal end than a position P2 of the wire insertion opening 131o. The position P1 of the medical liquid supply port 133o is also disposed closer to the distal end than a position P3 of the distal end of the wire 80 in maximal winding-up of the wire 80 to the wire winding part 511 of the wire moving device 50, which is described later. The blade parts 132 are two blade-shaped members extending from a lateral face of the first extension part 131 in the ±Y-axis direction, in the vicinity of the proximal end of the first extension part 131. The blade part 132 is used when a practitioner grips the connector 13. Members of at least portions of the first extension part 131, the blade part 132, and the second extension part 133, which configure the connector 13, may be integrally configured.

The hollow shaft 11 preferably has anti-thrombogenicity, flexibility, biocompatibility, and can be formed of a resin material, a metallic material, or the like. Examples of the resin materials to be employed can include a polyamide resin, a polyolefin resin, a polyester resin, a polyurethane resin, a silicon resin, a fluororesin. Examples of the metallic materials to be employed can include stainless steel such as SUS 304, Ni-Ti alloy, and cobalt-chromium alloy. The needle part 12 preferably has anti-thrombogenicity and biocompatibility, and can be formed of, e.g., a metallic material, including stainless steel such as SUS 304, Ni-Ti alloy, and cobalt-chromium alloy. The connector 13 can be formed of a resin material, such as polyurethane, polypropylene, or hard polyvinyl chloride.

### A-3. Detailed Configuration of Wire 80:

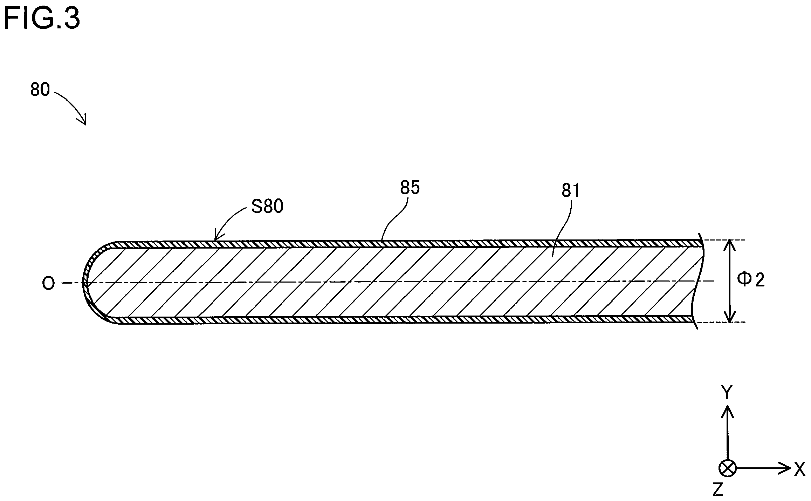

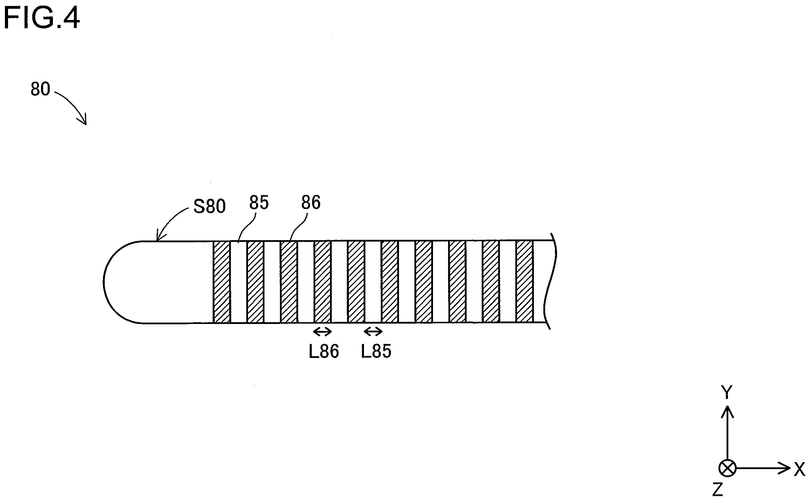

A configuration of the wire 80 will be described in detail with use of Fig. 3 and Fig. 4. Fig. 3 and Fig. 4 are explanatory diagrams illustrating a configuration in the distal end part of the wire 80. Fig. 3 depicts an XY cross-sectional configuration in the distal end part of the wire 80. Fig. 4 depicts an exterior configuration as seen in the Z-axis direction of the wire 80.

As shown in Fig. 3, the wire 80 has an outer surface S80, and includes a core shaft 81, and a coating part 85. The core shaft 81 is a long-shaped member extending along the axis line O, and has an approximately constant outer diameter from the distal end to the proximal end. The YZ cross-sectional shape of the core shaft 81 is approximately circular. The coating part 85 is a coated layer applied on the surface of the core shaft 81. The YZ cross-sectional shape of the wire 80 is approximately circular as similar to the core shaft 81. The core shaft 81 preferably has anti-thrombogenicity, flexibility, and biocompatibility, and can be formed of, e.g., a metallic material, including stainless steel such as SUS 304, and Ni-Ti alloy. The coating part 85 can be formed of PTFE (polytetrafluoroethylene), nylon, and a urethane-based resin. The wire 80 in the embodiment has an approximately constant outer diameter Φ2 from the distal end to the proximal end. The outer diameter Φ2 of the wire 80 is approximately the same (clearance: 100 pm or less) as the inner diameter Φ1 of the lumen 10L of the catheter 10 (see Fig. 2). The outer diameter Φ2 of the wire 80 is preferably 80% or more of the inner diameter Φ1 of the lumen 10L of the catheter 10.

As shown in Fig. 4, the wire 80 has a marker part 86 on the outer surface S80. In other words, the outer surface S80 consists of a surface exposing the coating part 85 and a surface applied with the marker part 86. The marker part 86 is applied with a prescribed width at a prescribed interval on the coating part 85. In the embodiment, the marker part 86 is applied with a width of 1 mm at an interval of 1 mm on the coating part 85. That is, in Fig. 4, both of a width L85 of a part exposing the coating part 85 and a width L86 of a part applied with the marker part 86 are 1 mm. The marker part 86 can be formed, e.g., by applying a prescribed material on the coating part 85. The prescribed material has chemical resistance, and is not particularly limited as long as it is a material having a wavelength of reflected light different from a wavelength of that in a material forming the coating part 85; for example, the material can be a material having a color different from that of a material forming the coating part 85. Such material causes a wavelength absorbed by a surface exposing the coating part 85 to differ from a wavelength absorbed by a surface applied with the marker part 86, thus providing reflected light with a different wavelength. The marker part 86 may be formed, e.g., by attaching a metallic seal formed of such material.

### A-4. Configuration of Wire moving device 50 and Storing part 60:

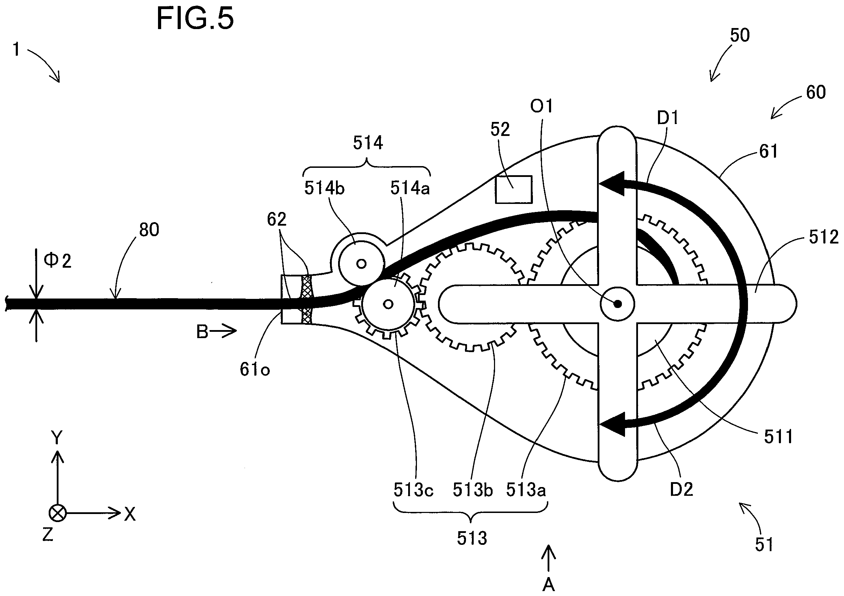

Fig. 5 is an explanatory diagram illustrating a configuration of the wire moving device 50 and the storing part 60. The wire moving device 50 moves the wire 80 forward inside the lumen 10L from the proximal end to the distal end side in accordance with a control signal, thereby ejecting a predetermined amount of medical liquid from the distal end of the hollow shaft 11. In the embodiment, the wire moving device 50 includes a wire driver part 51, a distance detection sensor 52, and a CPU built-in case 53. Note that Fig. 5 omits depiction of the CPU built-in case 53, which is described later, for convenience. The storing part 60 is connected to the proximal end part of the catheter 10, and contains a portion of the wire moving device 50 and a portion of the proximal end side of the wire 80. In the embodiment, the storing part 60 includes a housing 61 and a valve member 62.

The housing 61 is a case containing a portion of the wire moving device 50 (more particularly, a portion of the wire driver part 51 (the wire winding part 511, the gear 513, a wire extrusion part 514, described later) and the distance detection sensor 52) and the valve member 62 inside (in the inner part). Fig. 1 and Fig. 5 shows the housing 61 only by a contour as a transparent member, and indicates each member contained in the housing 61 by a solid line, for convenience of depiction.

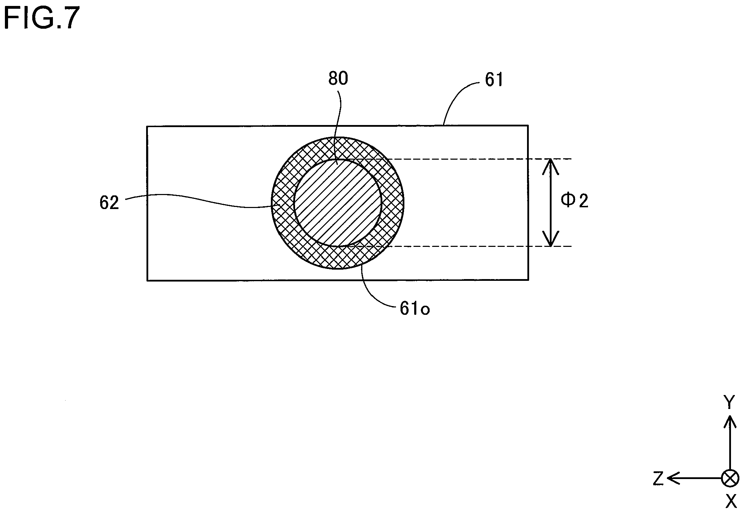

The valve member 62 is disposed between the wire driver part 51 (more particularly, the wire extrusion part 514, described later) and an opening 61o of the housing 61 as shown in Fig. 5, and is a member for regulating migration of medical liquid into the housing 61. Fig. 7 is an explanatory diagram illustrating of a configuration of the valve member 62. Fig. 7 depicts the housing 61, the valve member 62, and the wire 80 as seen in the B direction in Fig. 5. Note that the X-, Y-, and Z-axes in Fig. 7 correspond to the X-, Y-, and Z-axes in Fig. 1, respectively. As shown in Fig. 7, the valve member 62 surrounds the wire 80 between the wire extrusion part 514 and the opening 61o.

The wire driver part 51 is used for achieving forward movement of the wire 80 to the lumen 10L of the catheter 10, and backward movement of the wire 80 from the lumen 10L. More particularly, the wire driver part 51 moves the wire 80 forward by sending the wire 80 to the lumen 10L and moves the wire 80 backward by winding up the wire 80 from the lumen 10L. The wire driver part 51 has the wire winding part 511, the handle part 512, the gear 513, and the wire extrusion part 514.

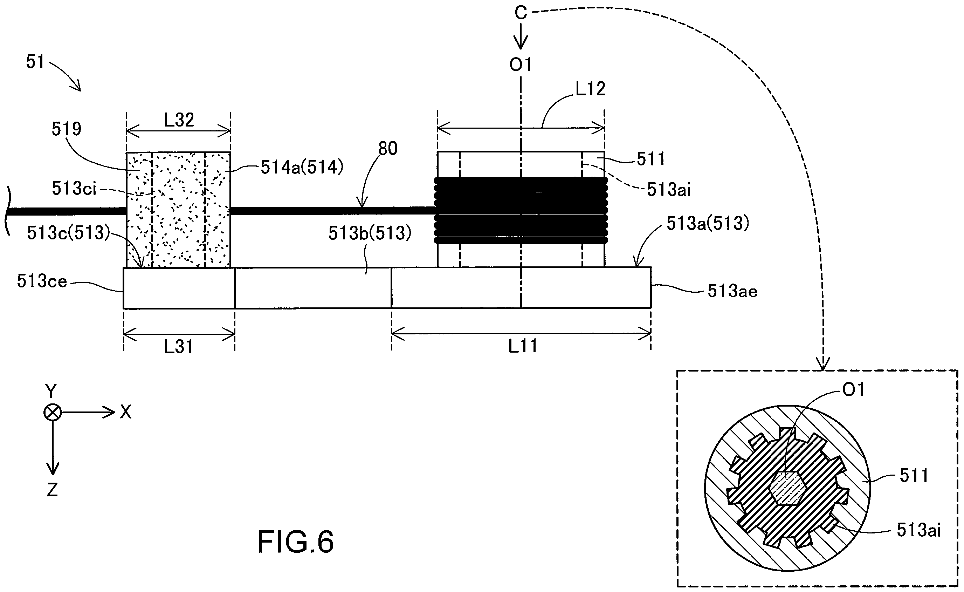

Fig. 6 is an explanatory diagram illustrating a configuration of the wire winding part 511, the gear 513, and the wire extrusion part 514. Fig. 6 depicts the wire winding part 511, the gear 513, the wire extrusion part 514, and the wire 80 as seen in the A direction in Fig. 5. The dashed-lined box in Fig. 6 shows a rotation shaft O1, the wire winding part 511, and a first gear 513a as seen in the C direction in Fig. 6. Note that the X-, Y-, and Z-axes in Fig. 6 correspond to the X-, Y-, and Z-axes in Fig. 1, respectively.

As shown in Fig. 5, the gear 513 includes the first gear 513a, a second gear 513b, and a third gear 513c. As shown in Fig. 6, the first gear 513a is a projected gear, and has outer teeth 513ae and inner teeth 513ai. Similarly, the third gear 513c is a projected gear, and has outer teeth 513ce and inner teeth 513ci. The first gear 513a is connected to the handle part 512 via the rotation shaft O1. The second gear 513b engages with each of the outer teeth 513ae of the first gear 513a and the outer teeth 513ce of the third gear 513c, and transmits, to the third gear 513c, a torque transmitted from the handle part 512 to the first gear 513a. A reference circle diameter L11 of the outer teeth 513ae of the first gear 513a is larger than a reference circle diameter L31 of the outer teeth 513ce of the third gear 513c (see Fig. 6). In other words, the first gear 513a has a larger number of the outer teeth 513ae than the number of the outer teeth 513ce of the third gear 513c. In the embodiment, a reference circle diameter of a gear means the average of a tooth tip circle diameter and a tooth bottom circle diameter.

The wire winding part 511 sends and winds up the wire 80 with a length corresponding to a rotation amount of the handle part 512. More particularly, the wire winding part 511 is an approximately hollow-cylindrical member fit to the inner teeth 513ai of the first gear 513a, and rotates corresponding to rotation of the first gear 513a (see the lower part in Fig. 6). The outer diameter of the wire winding part 511 is L12 (see Fig. 6). Around the wire winding part 511, a portion of the proximal end side of the wire 80 is wound beforehand. The proximal end part of the wire 80 may or may not be fixed to the wire winding part 511. The wire winding part 511 and the first gear 513a may be formed integrally.

The wire extrusion part 514 extrudes, toward the lumen 10L, the wire 80 sent from the wire winding part 511. More particularly, the wire extrusion part 514 includes a first roller 514a and a second roller 514b, and holds and extrudes the wire 80 by the first roller 514a and the second roller 514b. The first roller 514a is an approximately hollow-cylindrical member fit to the inner teeth 513ci of the third gear 513c, and rotates corresponding to rotation of the third gear 513c. In other words, the first roller 514a rotates corresponding to rotation of the handle part 512. The outer diameter of the first roller 514a is L32 (see Fig. 6). The lateral face of the first roller 514a is coated with a coating part 519. The first roller 514a and the third gear 513c may be formed integrally. The second roller 514b is an approximately hollow-cylindrical or approximately solid-cylindrical member disposed adjacent to the first roller 514a, and rotates corresponding to rotation of the first roller 514a. In other words, the second roller 514b rotates corresponding to rotation of the handle part 512. The lateral face of the second roller 514b is coated with the coating part 519 as similar to the first roller 514a.

As shown in Fig. 1 and Fig. 5, in the wire 80, a portion of the proximal end side is wound around the wire winding part 511, and a portion of the distal end side passes through the opening 61o of the housing 61 and exposes outside. The wire 80 is held by first roller 514a and the second roller 514b between the wire winding part 511 and the opening 61o. The wire 80 is surrounded by the valve member 62 between the wire extrusion part 514 and the opening 61o (see Fig. 7).

The handle part 512 is connected to the wire winding part 511. More particularly, the handle part 512 is a grip disposed outside the housing 61, and rotatable in each of a first direction D1 and a second direction D2, indicated by arrows, with centering the rotation shaft O1. The rotation shaft O1 of the handle part 512 extends inside the housing 61, and is connected to the gear 513 (first gear 513a) as described above. As described above, the rotation shaft O1 transmits, to the gear 513, a torque applied to the handle part 512.

The housing 61, the handle part 512, the wire winding part 511, the gear 513, and the wire extrusion part 514 as described above can be formed of a resin material such as polyurethane, polypropylene, or hard polyvinyl chloride. The housing 61, the handle part 512, the wire winding part 511, the gear 513, and the wire extrusion part 514 may be formed of the same material, or of materials different at least partially. The valve member 62, and the coating part 519 of the wire extrusion part 514 can be formed of, e.g., an elastic body such as silicon rubber or urethane rubber.

The distance detection sensor 52 detects an advance distance D of the wire 80. In the embodiment, the distance detection sensor 52 emits laser light to the wire 80, and detects an advance distance D of the wire 80 by reflection of the emitted laser light. The distance detection sensor 52 may be a sensor capable of detecting an advance distance D of the wire 80, and a distance detection sensor conventionally known can be employed. In the embodiment, the distance detection sensor 52 includes, e.g., a light emitter part that emits laser light to the wire 80, a light receiver part that receives reflected light produced by reflection of the emitted laser light by the wire 80, and a sender part that sends information on a wavelength of the reflected light thus received to a driving control device 533, which is described later. The light emitter part has a light source that generates laser light set in a wavelength range such as infrared. The light receiver part receives reflected light from the wire 80, as well as acquires wavelength information of the reflected light thus received. The sender part sends, to the driving control device 533, change information of a wavelength of the reflected light thus acquired in the presence of change equivalent to or more than a prescribed threshold in the wavelength, and wavelength information on the reflected light thus acquired.

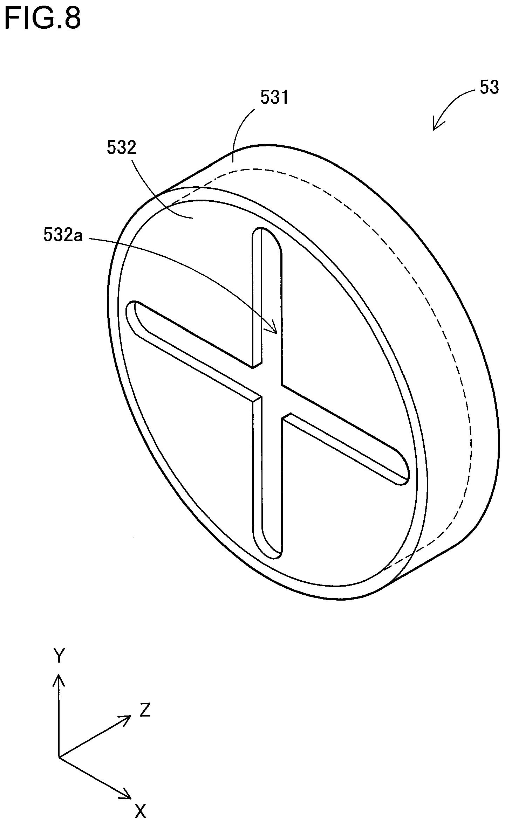

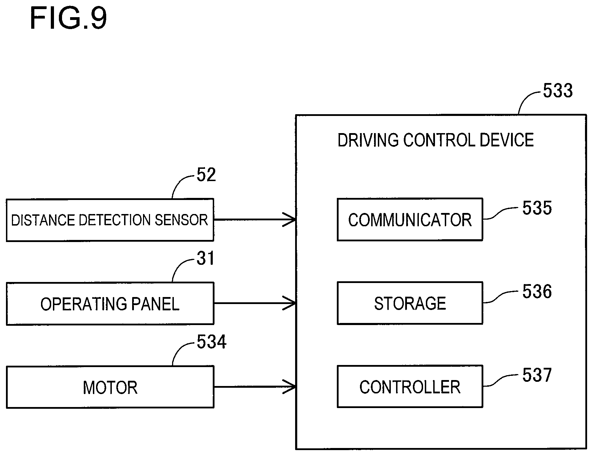

The CPU built-in case 53 is a hollow-cylindrical member to be fit to the handle part 512. Fig. 8 is an explanatory diagram illustrating a configuration of the CPU built-in case 53. Fig. 8 shows a perspective view of the CPU built-in case 53. The X-, Y-, and Z-axes in Fig. 8 correspond to the X-, Y-, and Z-axes in Fig. 1, respectively. More particularly, the CPU built-in case 53 includes an outer case 531, an inner rotating part 532, the driving control device 533 (see Fig. 9), and a motor 534 (see Fig. 9). That is, the CPU built-in case 53 (more particularly, the driving control device 533) is removably configured with the wire driver part 51.

The outer case 531 is a case that contains and protects the inner rotating part 532. In the embodiment, the outer case 531 includes the driving control device 533 and the motor 534 inside. The outer case 531 also has an operating panel 31 (see Fig. 9) on the outer surface.

The motor 534 functions as a power to rotate the inner rotating part 532.

The inner rotating part 532 is contained in the outer case 531, and has a cavity part 532a to be fit to the handle part 512, on a surface to face the handle part 512 (see Fig. 8). The inner rotating part 532 is electrically connected to the outer case 531. The inner rotating part 532 thus rotates in a circumferential direction by power from the motor 534. In other words, the handle part 512 fit to the inner rotating part 532 rotates in each of the first direction D1 and the second direction D2 as described above by rotation of the inner rotating part 532. The rotation of the inner rotating part 532 is controlled by the driving control device 533.

The operating panel 31 is an interface for inputting each piece of information for the driving control device 533. The operating panel 31 can be implemented in any aspect such as a touch panel or an operation button. Examples of the information for the driving control device 533 include a total dose of medical liquid Vt, a dose per administration V, and an administration start command for medical liquid. A total dose Vt is a total volume of medical liquid to be administered to a position of interest in the body of a patient, particularly, a total volume of medical liquid filled in the lumen 10L of the catheter 10 (in other words, contained in the catheter 10). A dose per administration V (hereinafter also referred to as "dose V") is an amount of medical liquid to be administered in a single puncture of the needle part 12 to the position of interest described above in a total dose Vt. An administration start command for medical liquid is an operation start command for the driving control device 533, more particularly, a sending command for the wire 80. Other examples of each piece of the information described above include an administration rate of medical liquid, a radius r of the wire 80 (i.e., an outer diameter of the wire 80 Φ2/2), pitch information of the marker part 86, and an operation termination command for the driving control device 533. Each piece of the information thus input is appropriately sent to the driving control device 533.

The driving control device 533 executes a process of terminating operation of the wire driver part 51 upon determining that an advance distance of the wire 80 derived based on a detection result of the distance detection sensor 52 is a distance corresponding to a predetermined dose of medical liquid. The driving control device 533 is an example of a control device within the scope of the claims.

The driving control device 533 will be outlined with use of Fig. 9. Fig. 9 depicts an example of a configuration of the driving control device 533 in the embodiment. As shown in Fig. 9, the driving control device 533 has a communicator 535, a storage 536, and a controller 537.

The communicator 535 receives information sent from the sender part of the distance detection sensor 52, and stores the information in the storage 536, which is described later.

The storage 536 stores, e.g., each piece of information input via the operating panel 31, information received at the communicator 535 from the distance detection sensor 52, a numerical formula for calculating an advance distance D of the wire 80, a predetermined advance speed of the wire 80, and information on the marker part 86 applied to the wire 80. Examples of the numerical formulae described above include a numerical formula for calculating an advance distance D of the wire 80 based on a dose of medical liquid and the diameter of the wire 80. Examples of information on the marker part 86 include pitch information of the marker part 86, more particularly, the width L85 of a part exposing the coating part 85 and the width L86 of a part applied with the marker part 86.

The controller 537 includes a function for executing a prescribed function by a code, instruction, or the like in a program, and is, e.g., a central processing unit (CPU). The controller 537 may also be e.g., a microprocessor, a multiprocessor, or the like. The controller 537 can execute, e.g., an information acquisition process, a first calculation process, a setting process, a command acquisition process, a medical liquid administration process, a wire retraction process, which are described below.

As the information acquisition process, the controller 537 acquires each piece of information input via the operating panel 31, such as a total dose of medical liquid Vt and a dose per administration V. In the embodiment, other pieces of information are acquired, such as an administration rate of medical liquid, a radius r of the wire 80 (i.e., an outer diameter of the wire 80 Φ2/2), and pitch information of the marker part 86.

As the first calculation process, the controller 537 calculates the number of divided administration Tt and a distance d to move the wire 80 forward (hereinafter referred to as "advance distance d of the wire 80"), based on the total dose Vt and the dose V acquired at the information acquisition process. The number of divided administration Tt is the number of administration required for administering the total dose Vt when the dose V is administered for every single puncture made by the needle part 12, and is calculated by the formula: (total dose Vt)/(dose V). An advance distance d of the wire 80 is a distance to move the wire 80 forward for administration of the dose V, and is calculated by the formula: (dose V)/(n × radius r² of the wire 80).

As the setting process, the controller 537 sets "1" to the current number of administration T.

As the command acquisition process, the controller 537 acquires an administration start command input via the operating panel 31.

As the medical liquid administration process, the controller 537 executes medical liquid administration to a position of interest in the body of a patient based on the administration start command acquired at the command acquisition process.

The medical liquid administration process can include, e.g., a drive start command process, a determination process, a second calculation process, a comparison process, a drive termination command process, which are described below.

As the drive start command process, the controller 537 drives the motor 534 based on the administration start command (a sending command of the wire 80) input via the operating panel 31, thereby rotating the inner rotating part 532 in the first direction D1. Along with the rotation of the inner rotating part 532 in the first direction D1, the handle part 512 also rotates in the first direction D1. That is, the drive start command process sends the wire 80 from the wire winding part 511.

As the determination process, the controller 537 monitors an advance distance D of the wire 80 with reference to wavelength information of reflected light stored in the storage 536, compares a wavelength of reflected light of the wire 80 received in the communicator 535 to a wavelength received immediately before, and determines whether the difference (hereinafter also referred to as "difference of wavelength") is equivalent to or more than a prescribed threshold or not. When the difference of wavelength is equivalent to or more than the prescribed threshold, determination can be made that a boundary between a part exposing the coating part 85 and a part applied with the marker part 86 in the outer surface S80 of the wire 80 has passed through the distance detection sensor 52.

As the second calculation process, the controller 537 calculates an advance distance D of the wire 80 based on pitch information of the marker part 86 stored in the storage 536. For example, the controller 537 can calculate the sum of advance distances D of the wire 80 by adding the width L85 of a part exposing the coating part 85 or the width L86 of a part applied with the marker part 86 in the wire 80 to an advance distance D of the wire 80, every time when the difference of wavelength described above is determined as equivalent to or more than the prescribed threshold.

As the comparison process, the controller 537 compares the advance distance d of the wire 80 calculated at the first calculation process to the advance distance D of the wire 80 calculated at the second calculation process.

As the drive termination command process, the controller 537 terminates rotation of the inner rotating part 532 (the handle part 512), when the advance distance d of the wire 80 is identical with the advance distance D at the comparison process.

In the embodiment, the driving control device 533 further executes a wire retraction process. As the wire retraction process, after the drive termination command process terminates rotation of the inner rotating part 532, and then upon determination that all of the number of divided administration Tt has executed, the controller 537 drives the motor 534, thereby rotating the inner rotating part 532 in the second direction D2. Along with the rotation of the inner rotating part 532 in the second direction D2, the handle part 512 also rotates in the second direction D2. That is, the wire 80 is wound up to the wire winding part 511.

### A-5. Processing Flow of Driving Control Device 533:

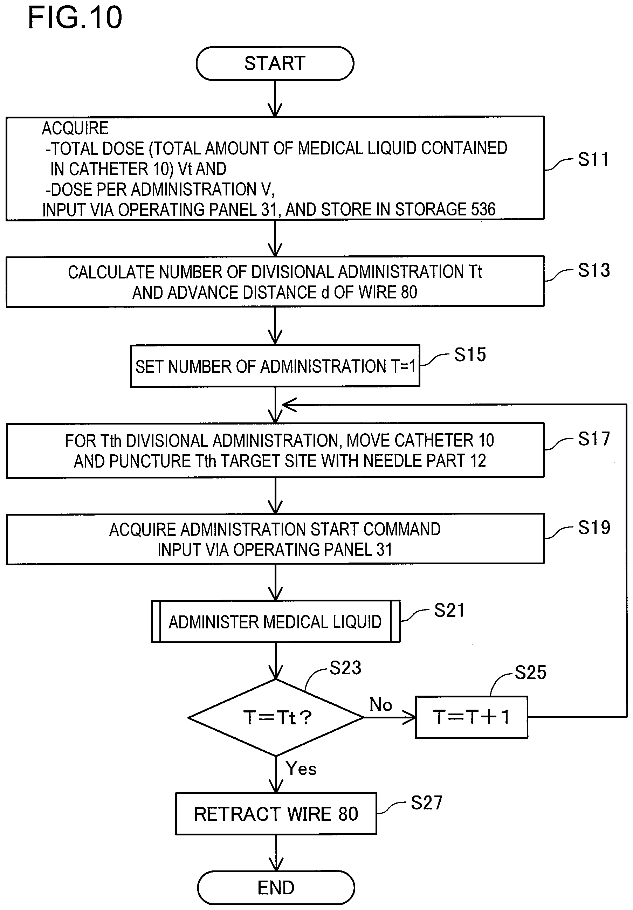

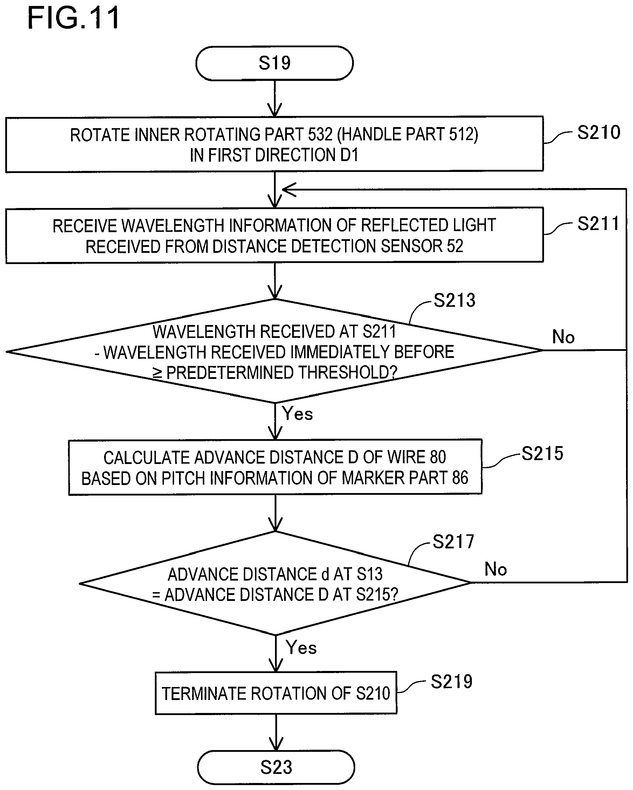

A processing flow of the driving control device 533 will be outlined with use of Fig. 10 and Fig. 11. Fig. 10 is a flowchart depicting an example of a processing flow of the driving control device 533 in the embodiment, and Fig. 11 is a flowchart depicting an example of a processing flow of step S21 in the embodiment. The processing flow of the driving control device 533 in the embodiment has, e.g., step S11-step S27.

At step S11, the controller 537 acquires each piece of information input via the operating panel 31, and stores the information in the storage 536 (information acquisition process). Examples of each piece of the information include a total dose of medical liquid Vt and a dose per administration V. Other examples of each piece of the information described above include an administration rate of medical liquid, a radius r of the wire 80 (i.e., an outer diameter of the wire 80 Φ2/2), and pitch information of the marker part 86. In the embodiment, assume that the storage 536 stores a total dose Vt of "5 mL", a dose V of "1 mL", and a radius r of the wire 80, for example.

At step S13, the controller 537 calculates the number of divided administration Tt and an advance distance d of the wire 80 with reference to the storage 536 (the first calculation process). In the embodiment, assume that "five times" is calculated as the number of divided administration Tt, for example.

At step S15, the controller 537 sets "1" to the current number of administration T (setting process).

At step S17, in order to execute the Tth round of divisional administration (the Tth divisional administration), a practitioner moves the catheter 10, and punctures a position of interest (particularly, the Tth target site in the position of interest) with the needle part 12. At step S17 executed subsequent to step S15, puncture of the needle part 12 to a first target site is made for executing a first divisional administration.

At step S19, the controller 537 acquires an administration start command input via the operating panel 31 (command acquisition process).

At step S21, medical liquid administration is executed based on the administration start command in step S19. In particular, at step S21, the first divisional administration is executed (medical liquid administration process).

A processing flow in step S21 will be described in detail with use of Fig. 11. At step S210, the controller 537 drives the motor 534 based on the administration start command (a sending command of the wire 80) input via the operating panel 31, thereby rotating the inner rotating part 532 in the first direction D1 (drive start command process).

At step S211, the communicator 535 receives, from the sender part of the distance detection sensor 52, wavelength information of reflected light received by the light receiver part of the distance detection sensor 52, and stores the information in the storage 536.

At step S213, the controller 537 monitors an advance distance D of the wire 80 with reference to wavelength information of reflected light stored in the storage 536, compares the wavelength received at step S211 to a wavelength received immediately before, and determines whether the difference of wavelength is equivalent to or more than a prescribed threshold or not (determination process). When the difference of wavelength is equivalent to or more than the prescribed threshold (step S213: Yes), the flow proceeds to step S215. When determination at step S213 is "No", the flow returns to step S211.

At step S215, the controller 537 calculates an advance distance D of the wire 80 based on pitch information of the marker part 86 stored in the storage 536 (second calculation process). As described above, in the embodiment, both of the width L85 and the width L86 are 1 mm, and the controller 537 thus adds 1 mm to the advance distance D of the wire 80 and calculates the sum of the advance distance D of the wire 80, every time when the difference of wavelength described above is determined as equivalent to or more than the prescribed threshold.

At step S217, the controller 537 compares the advance distance d of the wire 80 calculated at step S13 to the advance distance D of the wire 80 calculated at step S215 (comparison process). When both are identical (step S217: Yes), the flow proceeds to step S219. When determination at step S217 is "No", the flow returns to step S211.

At step S219, the controller 537 terminates rotation of the inner rotating part 532 (handle part 512) in step S210 (drive termination command process). In particular, the controller 537 terminates drive of the motor 534. The foregoing processes completes execution of the Tth divisional administration to the Tth target site (e.g., the first divisional administration to the first target site).

Return to Fig. 10, step S17 to step S21 are executed for all of the number of divided administration Tt calculated at step S13 (step S23, step S25). At step S23, when all of the number of divided administration Tt are determined to be completed (step S23: Yes), the flow proceeds to step S27. In the embodiment, the number of divided administration Tt is "5" as described above, and thus the processes of step S17 to step S21 are repeated five times. That is, for the first to fifth target sites, the first to fifth divisional administrations are executed respectively. All of the first to fifth target sites are sites present in the range of the position of interest, and can be, e.g., sites individually present in different locations.

At step S27, the wire 80 is retracted (wire retraction process). The foregoing processes can complete administration of a total dose Vt of medical liquid to a position of interest (e.g., affected area) and insertion and evulsion of the wire 80.

### A-6. Action of Medical Liquid Injection Device 1

According to the medical liquid injection device 1 in the embodiment, inclusion of the configuration described above allows the wire moving device 50 to automatically or manually move the wire 80 forward and backward in the lumen 10L of the catheter 10. In particular, as shown in Fig. 5, when the handle part 512 of the wire moving device 50 is automatically or manually rotated in the first direction D1, a torque applied to the handle part 512 is transmitted to the first gear 513a and the wire winding part 511 via the rotation shaft O1. This rotates the wire winding part 511 in the first direction D1 to send the wire 80 with a length corresponding to a rotation amount of the handle part 512, toward the lumen 10L (the wire 80 moves forward inside the lumen 10L). When the handle part 512 of the wire moving device 50 is rotated in the second direction D2, the wire winding part 511 rotates in the second direction D2 similarly, and the wire 80 with a length corresponding to a rotation amount of the handle part 512 is wound up from the lumen 10L (the wire 80 moves backward inside the lumen 10L).

Rotation of the first gear 513a along with rotation of the handle part 512 is transmitted to the third gear 513c via the second gear 513b. This leads to rotation of the first roller 514a and the second roller 514b, thereby extruding the wire 80 held by the first roller 514a and the second roller 514b toward the lumen 10L. Note that as shown in Fig. 6, the ratio of the reference circle diameter L31 of the third gear 513c to the outer diameter L32 of the first roller 514a (L31/L32) is less than the ratio of the reference circle diameter L11 of the first gear 513a to the outer diameter L12 of the wire winding part 511 (L11/L12). Therefore, for the same rotation amount of the handle part 512, the length of the wire 80 to be extruded by the first roller 514a and the second roller 514b is larger than the length of the wire 80 to be sent by the wire winding part 511. In other words, the first roller 514a and the second roller 514b extrude the wire 80 in a direction to the lumen 10L, with some idle running and with holding the wire 80.

Furthermore, since lateral faces of the first roller 514a and the second roller 514b (faces holding the wire 80) is coated with the coating part 519 formed of an elastic body, the first roller 514a and the second roller 514b can hold the wire 80, and extrude the wire 80 in a direction of the lumen 10L by frictional force.

### A-7. Method of Use of Medical Liquid Injection Device 1:

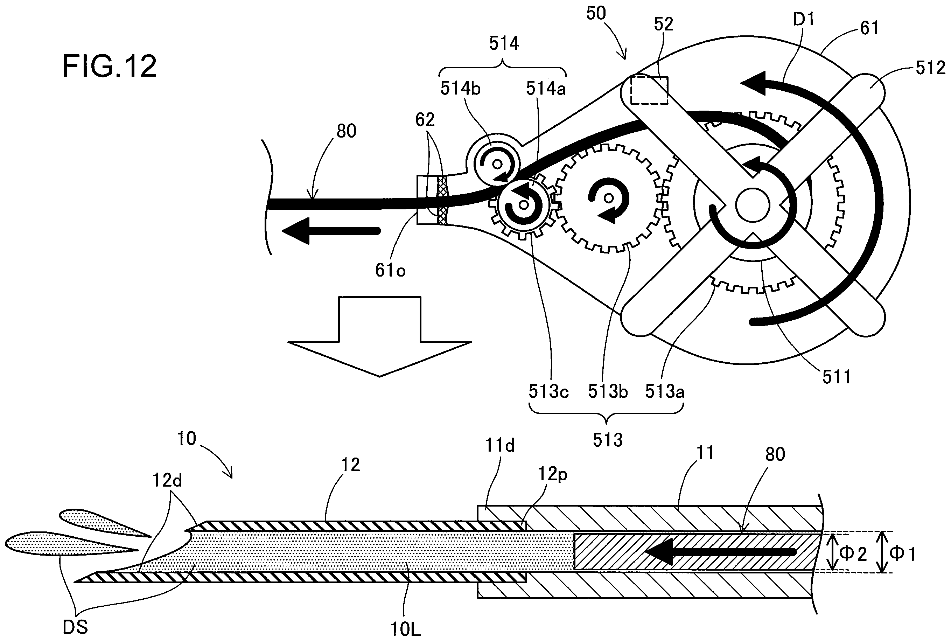

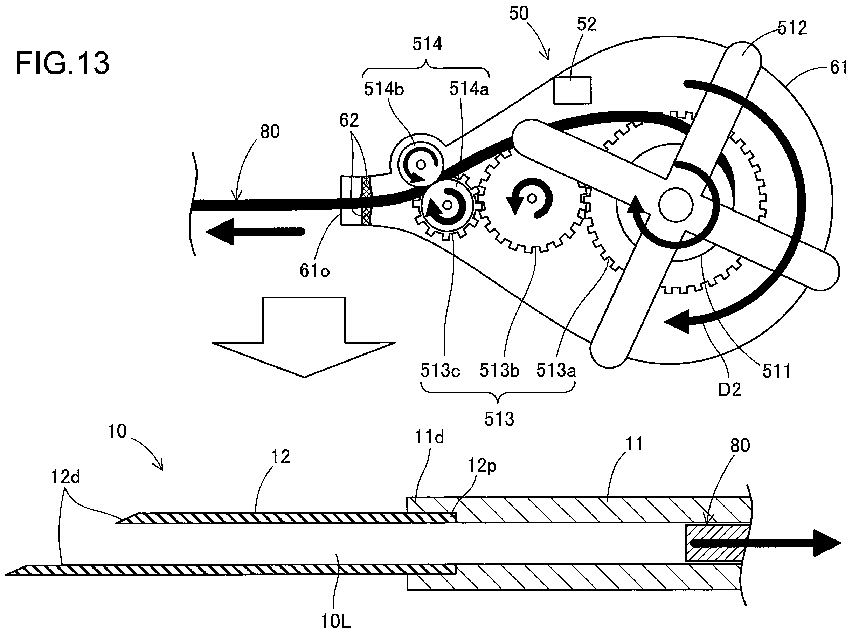

Fig. 12 illustrates forward movement of the wire 80 and ejection of medical liquid. Fig. 13 illustrates backward movement of the wire 80. Fig. 12 and Fig. 13 depicts the wire moving device 50 in the upper part, and a portion of the distal end side of the catheter 10 in the lower part. A method of using the medical liquid injection device 1 will be described with use of Fig. 1, Fig. 12, and Fig. 13.

In use of the medical liquid injection device 1 with an endoscope, a practitioner inserts the catheter 10 into the endoscope, and moves the needle part 12 of the catheter 10 to a position of interest in the body of a patient (a position to receive administration of medical liquid). Then the practitioner attaches, to the wire insertion opening 131o of the catheter 10, the storing part 60 equipped with the wire moving device 50 (see Fig. 1). At that time, the attachment of the storing part 60 is made while the distal end part of the wire 80 extruded from the opening 61o of the storing part 60 is inserted into the lumen 10L of the catheter 10. Then, the practitioner supplies medical liquid from the medical liquid supply port 133o of the catheter 10. For example, a syringe containing medical liquid is attached to the proximal end part of the second extension part 133, and medical liquid is supplied from the syringe. The medical liquid supplied via the second extension part 133 from the medical liquid supply port 133o is filled in the lumen 10L of the catheter 10. Then, the practitioner punctures the position of interest in the body of the patient with the needle part 12.

In this regard, when the medical liquid injection device 1 is used without an endoscope, the practitioner attaches, to the catheter 10, the storing part 60 equipped with the wire moving device 50, and inserts the catheter 10 into the living body lumen of the patient while medical liquid is filled in the lumen 10L of the catheter 10 beforehand. Then, the practitioner moves the needle part 12 of the catheter 10 to the position of interest in the body of the patient, and punctures the position of interest with the needle part 12.

The practitioner inputs, via the operating panel 31, each piece of information such as a total dose Vt of medical liquid to be administered to the patient, a dose per administration V, an administration rate of medical liquid, a radius r of the wire 80, pitch information of the marker part 86 (more particularly, the width L85 of a part exposing the coating part 85 of the wire 80 and the width L86 of a part applied with the marker part 86, or the like). Each piece of the information thus input is sent to the driving control device 533. The controller 537 of the driving control device 533 calculates the number of divided administration Tt and an advance distance d of the wire 80 based on each piece of the information described above.

At first, the first divisional administration is performed to the first target site in the position of interest. More particularly, after puncturing the first target site with the needle part 12, a practitioner inputs an administration start command for medical liquid via the operating panel 31. Upon input of the administration start command for medical liquid of the driving control device 533, the controller 537 of the driving control device 533 drives the motor 534, and rotates the inner rotating part 532 in the first direction D1 as shown in the upper part in Fig. 12. Then, the handle part 512 also rotates in the first direction D1 along with rotation of the inner rotating part 532, and the first gear 513a and the wire winding part 511 rotate in the first direction D1 along with rotation of the handle part 512, thereby sending the wire 80 to the lumen 10L of the catheter 10 (i.e., moving the wire 80 forward). Then, as shown in the lower part in Fig. 12, medical liquid DS filled in the lumen 10L of the catheter 10 is push out by the wire 80 moved forward, and the medical liquid DS having a volume corresponding to a portion of the wire 80 moved forward is ejected from the distal end of the needle part 12. That is, the medical liquid DS having an amount corresponding to rotation amount of the handle part 512 is ejected from the distal end of the needle part 12. Along with rotation of the first gear 513a, the second gear 513b rotates in a direction opposite to the first direction D1, the third gear 513c and the first roller 514a rotates in the first direction D1, and the second roller 514b rotates in a direction opposite to the first direction D1. This leads to extrusion of the wire 80 to the lumen 10L of the catheter 10.

During forward movement of the wire 80, the light emitter part of the distance detection sensor 52 emits laser light to the outer surface S80 of the wire 80, and the light receiver part receives reflected light from the wire 80. The sender part sends wavelength information of the reflected light received by the light receiver part to the driving control device 533. The controller 537 of the driving control device 533 terminates operation of the driving control device 533 based on wavelength information of the reflected light and an advance distance d of the wire 80, when the wire 80 reaches the advance distance d of the wire 80.

After injecting the medical liquid DS in the position of interest in the body of the patient (the first target site), the practitioner punctures a second target site in the position of interest with the needle part 12 in order to perform a second divisional administration at the second target site, and inputs an administration start command for medical liquid via the operating panel 31.

Upon execution of all of the number of divided administration Tt described above, the controller 537 retracts the wire 80. More particularly, the controller 537 drives the motor 534, and rotates the inner rotating part 532 in the second direction D2 as shown in the upper part in Fig. 13. Then, the handle part 512 also rotates in the second direction D2 along with rotation of the inner rotating part 532, and the first gear 513a and the wire winding part 511 rotate in the second direction D2 along with rotation of the handle part 512, thereby winding up the wire 80 from the lumen 10L of the catheter 10 (i.e., moving the wire 80 backward). Along with rotation of the first gear 513a, the second gear 513b rotates in a direction opposite to the second direction D2, the third gear 513c and the first roller 514a rotates in the second direction D2, and the second roller 514b rotates in a direction opposite to the second direction D2.

### A-8. Effects of First Embodiment:

As illustrated above, medical liquid injection device 1 in the embodiment includes the catheter 10 with the hollow shaft 11 having the lumen 10L formed inside to contain medical liquid, the wire 80 to be inserted into the lumen 10L, and the wire moving device 50. The wire moving device 50 moves the wire 80 forward inside the lumen 10L from a proximal end to a distal end side in accordance with a control signal from the driving control device 533. This causes a predetermined amount of medical liquid to be ejected from the distal end of the hollow shaft 11.

The medical liquid injection device 1 in the embodiment includes the wire 80 to be inserted into the lumen 10L to contain medical liquid, wherein the wire 80 is moved forward from the proximal end to the distal end side of the lumen 10L, thereby ejecting the medical liquid in the lumen 10L from the distal end of the hollow shaft 11. That is, the medical liquid injection device 1 in the embodiment pushes out the medical liquid in the lumen 10L by the wire 80, thereby ejecting from the distal end of the hollow shaft 11. This allows an amount of the medical liquid to be ejected to have the same amount as the volume of the wire 80, thus providing precise control of an injection amount of the medical liquid. The medical liquid injection device 1 in the embodiment can also push out the medical liquid in the lumen 10L by the wire 80, thereby allowing full use of the medical liquid in the lumen 10L. This enables prevention of waste of the medical liquid. The medical liquid injection device 1 in the embodiment also includes the wire moving device 50 that moves the wire 80 forward into the lumen 10L in accordance with a control signal from the driving control device 533. This allows the medical liquid injection device 1 in the embodiment to automatically execute forward movement of the wire 80 into a lumen 10L. Accordingly, the medical liquid injection device 1 in the embodiment enables automatic injection of medical liquid with precisely controlling an injection amount of the medical liquid to reduce waste of the medical liquid.

In the medical liquid injection device 1 in the embodiment, the catheter 10 has the hollow needle part 12 at the distal end of the hollow shaft 11. Thus, in the medical liquid injection device 1 in the embodiment, puncture can be made with the needle part 12 at a position of interest inside the body of a patient (e.g., affected area) when medical liquid is injected at the position of interest. Therefore, the medical liquid injection device 1 in the embodiment enables fixation of the needle part 12 to a position of interest inside the body of a patient, thus allowing precise injection of medical liquid at the position of interest, as well as injection of the medical liquid inside the position of interest.

In the medical liquid injection device 1 in the embodiment, the wire moving device 50 has the wire driver part 51 to move the wire 80 forward, the distance detection sensor 52 to detect an advance distance D of the wire 80, and the driving control device 533. The driving control device 533 also terminates operation of the wire driver part 51 upon determining that an advance distance of the wire 80 calculated based on a detection result of the distance detection sensor 52 is a distance corresponding to a predetermined amount. Thus, in terminating operation of the wire driver part 51, the medical liquid injection device 1 in the embodiment enables more precise termination of operation of the wire driver part 51 by using an advance distance D of the wire 80 calculated for a detection result of the distance detection sensor 52.

In the medical liquid injection device 1 in the embodiment, the wire moving device 50 has the wire driver part 51 removably configured with the driving control device 533. The medical liquid injection device 1 in the embodiment allows automatic forward movement of the wire 80 into the lumen 10L in a configuration with the driving control device 533 attached to the wire driver part 51, as well as manual forward movement of the wire 80 into the lumen 10L in a configuration with the driving control device 533 removed from the wire driver part 51. For example, in injection of medical liquid on the surface or inside (hereinafter referred to as "the surface or the like") of an affected area in the body of a patient, a practitioner can manually move the wire 80 forward to the lumen 10L, thereby obtaining a sense of the surface or the like of the affected area. The practitioner can recognize, based on this sense, whether the surface or the like of the affected area is suited for injection of medical liquid. For example, injection pressure of medical liquid in manually moving the wire forward to the lumen provides a sense of hardness of the surface or the like of the affected area, and then this sense can provide a basis to move a position for injecting medical liquid to another position in the affected area when the affected area has a certain hardness on the surface or the like (has hardness unsuitable for injecting medical liquid).

In the medical liquid injection device 1 in the embodiment, the wire driver part 51 is configured to move the wire 80 forward by sending the wire 80 to the lumen 10L and moves the wire 80 backward by winding up the wire 80 from the lumen 10L. The medical liquid injection device 1 in the embodiment allows the wire driver part 51 to move the wire 80 forward by sending the wire 80 to the lumen 10L and to move the wire 80 backward by winding up the wire 80 from the lumen 10L, thus ensuring forward and backward movement of the wire 80. In addition, since the wire 80 is wound up to be stored, a space required for storing the wire 80 (the storing part 60, described later) can be miniaturized.

In the medical liquid injection device 1 in the embodiment, the wire driver part 51 has the wire winding part 511 having winding of a portion of the proximal end side of the wire 80, and the handle part 512 connected to the wire winding part 511. The wire winding part 511 is configured to send and wind up the wire 80 in a length corresponding to a rotation amount of the handle part 512. The medical liquid injection device 1 in the embodiment allows sending and winding-up of the wire 80 by rotating the handle part 512, thus providing the medical liquid injection device 1 with a simple configuration.

The medical liquid injection device 1 in the embodiment includes the storing part 60 connected to the proximal end part of the catheter 10 and containing a portion of the proximal end side of the wire 80 and a portion of the wire driver part 51. The medical liquid injection device 1 in the embodiment includes the storing part 60 connected to the proximal end part of the catheter 10, storing a portion of the proximal end side of the wire 80, as well as storing a portion of the wire driver part 51 that enables forward and backward movement of the wire 80 inside the lumen 10L. This allows a practitioner to move the wire 80 forward and backward without directly touching the wire 80.

In the medical liquid injection device 1 in the embodiment, the wire moving device 50 further includes the wire extrusion part 514 to extrude, toward the lumen 10L, the wire 80 sent from the wire winding part 511, wherein the wire extrusion part 514 includes the first roller 514a and the second roller 514b to rotate corresponding to rotation of the handle part 512, and holds and extrudes the wire 80 by the first roller 514a and the second roller 514b. In the medical liquid injection device 1 in the embodiment, the wire moving device 50 includes the wire extrusion part 514 that extrudes, toward the lumen 10L, the wire 80 sent from the wire winding part 511, thus allowing the wire 80 to be securely pushed forward into the lumen 10L.

In the medical liquid injection device 1 in the embodiment, the storing part 60 further includes the valve member 62, which is disposed between the wire extrusion part 514 and the proximal end part of the catheter 10 and regulates migration of medical liquid in the lumen 10L into the storing part 60. The medical liquid injection device 1 in the embodiment enables preventing medical liquid in the lumen 10L from entering the storing part 60, and allows maintaining airtightness in the storing part 60.

In the medical liquid injection device 1 in the embodiment, the catheter 10 further includes the connector 13, which has the medical liquid supply port 133o for supplying medical liquid to the lumen 10L and the wire insertion opening 131o for inserting the wire 80 into the lumen 10L, wherein the medical liquid supply port 133o is connected to the proximal end part of the hollow shaft 11 and communicates with the lumen 10L. The medical liquid supply port 133o is disposed closer to the distal end than the distal end of the wire 80 in maximal winding-up of the wire 80 to the wire winding part 511 by rotation of the handle part 512. In the medical liquid injection device 1 in the embodiment, the medical liquid supply port 133o of the connector 13 is disposed closer to the distal end than the distal end of the wire 80 when the wire 80 is maximally wound up to the wire winding part 511. This allows supply of medical liquid from the medical liquid supply port 133o without inhibition by the wire 80, when the handle part 512 is rotated to maximally wind up the wire 80 to the wire winding part 511.

In the medical liquid injection device 1 in the embodiment, the connector 13 further includes the first extension part 131 extending in a longitudinal direction of the catheter 10, and the second extension part 133 extending from a lateral face of the first extension part 131 in the vicinity of the distal end of the first extension part 131 in a direction different from that of the first extension part 131. The medical liquid supply port 133o is also formed in the distal end part of the second extension part 133, and the wire insertion opening 131o is formed in the proximal end part of the first extension part 131. In medical liquid injection device 1 in the embodiment, the connector 13 includes the wire insertion opening 131o formed in the proximal end part of the first extension part 131, and the medical liquid supply port 133o formed in the distal end part of the second extension part 133 extending from a lateral face of the first extension part 131 in the vicinity of the distal end of the first extension part 131. This enables smooth insertion of the wire 80 into the wire insertion opening 131o and supply of medical liquid from the medical liquid supply port 133o.

In the medical liquid injection device 1 in the embodiment, the outer diameter of the wire 80 is approximately constant from the distal end to the proximal end. The medical liquid injection device 1 in the embodiment allows easy recognition of an amount of medical liquid to be ejected from the distal end of the hollow shaft 11 (or the needle part 12) corresponding to a length of the wire 80 to be moved inside the lumen 10L.

In the medical liquid injection device 1 in the embodiment, each of a cross-sectional shape of the lumen 10L and a cross-sectional shape of the wire 80 is approximately circular. In addition, the outer diameter Φ2 of the wire 80 is approximately the same as the inner diameter Φ1 of the lumen 10L. The medical liquid injection device 1 in the embodiment enables the wire 80 to push out medical liquid in the lumen 10L with maintaining slidability of the wire 80 inside the lumen 10L.

### B. Second Embodiment:

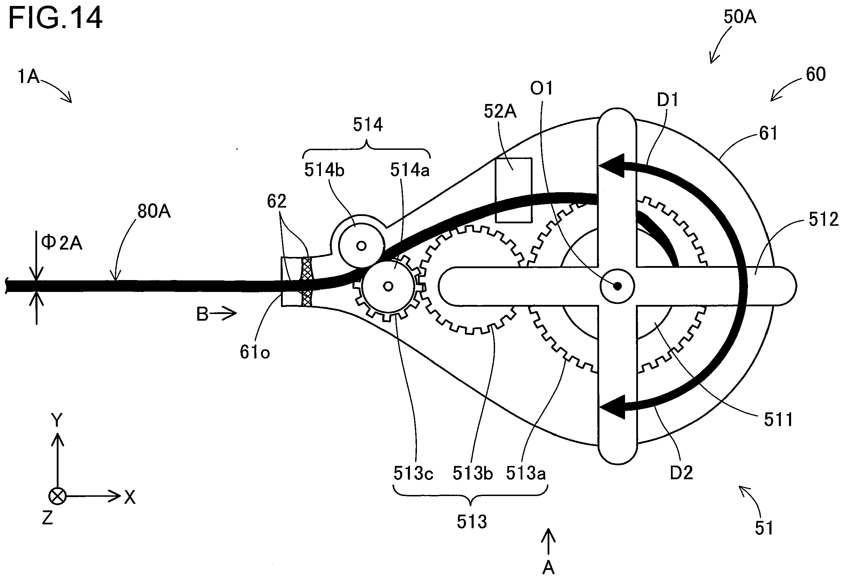

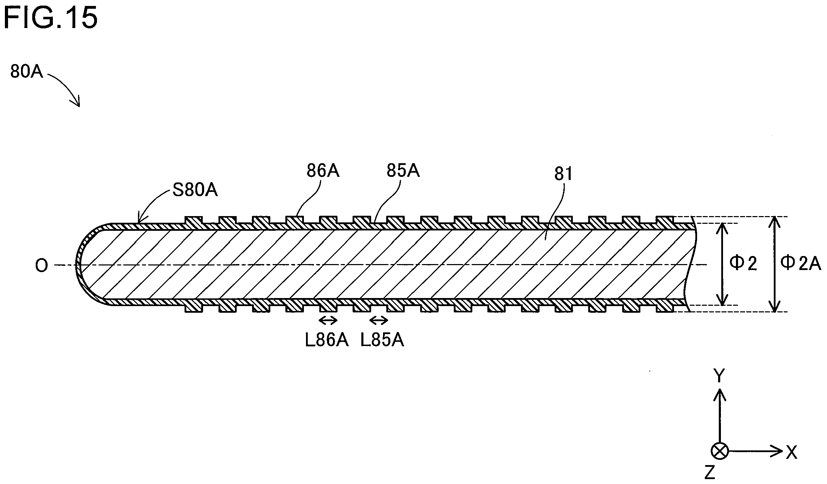

Fig. 14 is an explanatory diagram illustrating a configuration of a wire moving device 50A and the storing part 60 included in a medical liquid injection device 1A in a second embodiment. Note that Fig. 14 omits depiction of the CPU built-in case 53 for convenience. Fig. 15 is an explanatory diagram illustrating a configuration in the distal end part of a wire 80A. Fig. 15 depicts a XY cross-sectional configuration in the distal end part of the wire 80A. Hereinafter, in a configuration of the medical liquid injection device 1A in the second embodiment, description will be appropriately omitted for the same configuration as the medical liquid injection device 1 in the first embodiment, which is described above.

The medical liquid injection device 1A in the second embodiment includes the wire moving device 50A instead of the wire moving device 50, which is described in first embodiment, and wire 80A instead of the wire 80.

As shown in Fig. 15, the wire 80A includes the core shaft 81 and a coating part 85A. The coating part 85A is a coated layer applied on the surface of the core shaft 81 as similar to the coating part 85 in the first embodiment. The wire 80A has a marker part 86A formed in a projected shape on the coating part 85A, on an outer surface S80A. The marker part 86A is applied with a prescribed width at a prescribed interval on the coating part 85A. In the embodiment, the marker part 86A is formed with a width of 1 mm at an interval of 1 mm on the coating part 85A. That is, in Fig. 15, both of a width L85A of a part not forming the marker part 86A and a width L86A of the marker part 86A in the X-axis direction are 1 mm. The marker part 86A can be formed of, e.g., a material similar to that of the coating part 85A, on the coating part 85A. The wire 80A in the embodiment has an outer diameter Φ2 in a part not forming the marker part 86A, and an outer diameter Φ2A in a part forming the marker part 86A. The outer diameter Φ2A of the wire 80A is approximately the same (clearance: 100 pm or less) as the inner diameter Φ1 of the lumen 10L of the catheter 10 (see Fig. 2). The outer diameter Φ2A of the wire 80A is preferably 80% or more of the inner diameter Φ1 of the lumen 10L of the catheter 10.

The wire moving device 50A includes a distance detection sensor 52A instead of the distance detection sensor 52 described in the first embodiment. The distance detection sensor 52A detects an advance distance D of the wire 80A. In the embodiment, the distance detection sensor 52A is a pressure sensor, and can utilize a projected marker part 86A formed on the wire 80A. More particularly, the distance detection sensor 52A detects pressure that changes in transit of the marker part 86A, and sends change information of the pressure, detected pressure information, and the like to the driving control device 533.

The driving control device 533 executes a process of terminating operation of the wire driver part 51 upon determining that an advance distance of the wire 80A calculated based on a detection result of the distance detection sensor 52A is a distance corresponding to a predetermined dose of medical liquid. The medical liquid injection device 1A, which uses the distance detection sensor 52A and the wire 80A in the second embodiment as such, also provides an effect similar to that of the first embodiment.

### C. Third Embodiment:

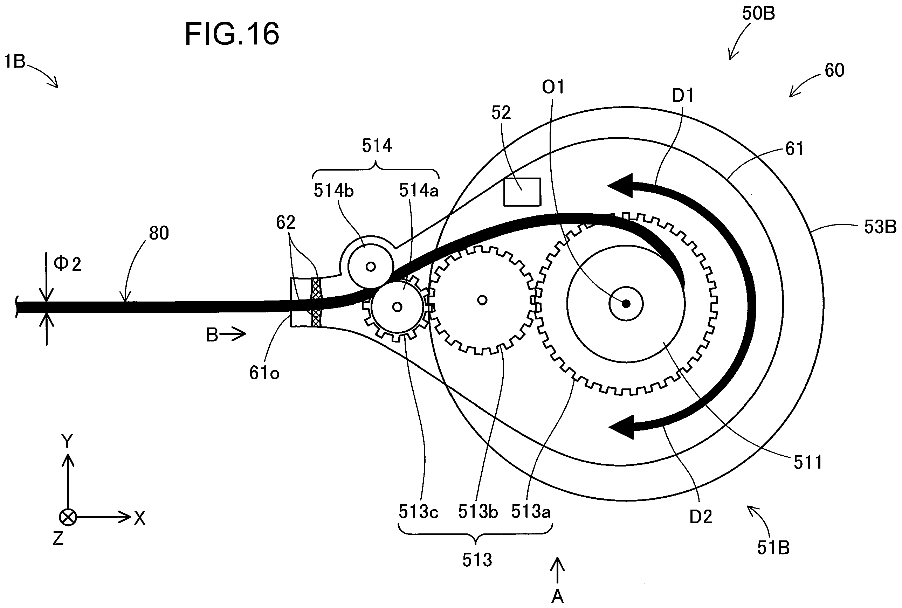

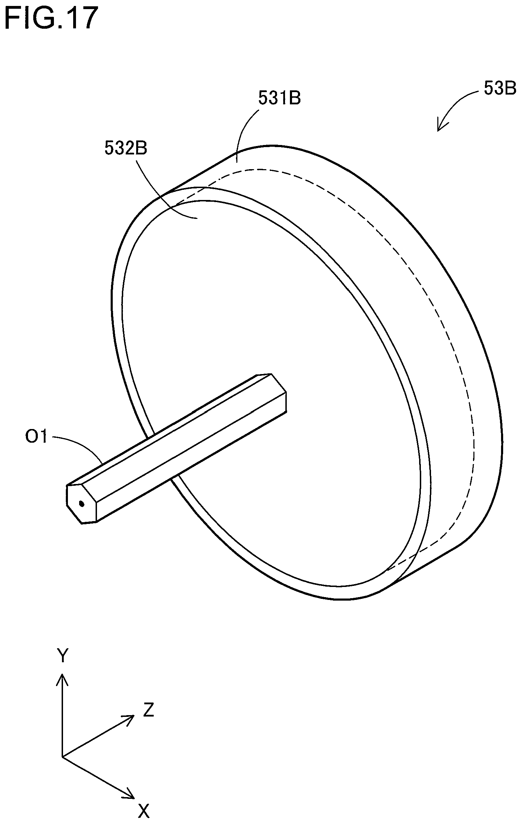

Fig. 16 is an explanatory diagram illustrating a configuration of a wire moving device 50B and the storing part 60 included in a medical liquid injection device 1B in a third embodiment. Fig. 17 is an explanatory diagram illustrating a configuration of a CPU built-in case 53B included in the wire moving device 50B in the third embodiment. Fig. 17 depicts a perspective view of the CPU built-in case 53B. Fig. 16 shows the CPU built-in case 53B only by a contour as a transparent member, and omits each member configuring the CPU built-in case 53B. The X-, Y-, and Z-axes in Fig. 16 and Fig. 17 correspond to the X-, Y-, and Z-axes in Fig. 1, respectively. Hereinafter, in the configuration of the medical liquid injection device 1B in the third embodiment, description will be appropriately omitted for the same configuration as the medical liquid injection devices 1 and 1A in the first embodiment and the second embodiment described above.

The medical liquid injection device 1B in the third embodiment includes the wire moving device 50B instead of the wire moving device 50 in the first embodiment.

As shown in Fig. 16, the wire moving device 50B includes a wire driver part 51B, the distance detection sensor 52, and a CPU built-in case 53B. The wire driver part 51B has the wire winding part 511, the gear 513, and the wire extrusion part 514. The CPU built-in case 53B includes an outer case 531B, an inner rotating part 532B, a rotation shaft O1, the driving control device 533, and the motor 534. In the embodiment, the CPU built-in case 53B (more particularly, the driving control device 533) is configured integrally with the wire driver part 51B. More particularly, in the CPU built-in case 53B in the embodiment, the rotation shaft O1 included in the inner rotating part 532B is connected to the wire winding part 511 (more particularly, the first gear 513a). As described above, drive of the motor transmits a torque applied to the inner rotating part 532B to the first gear 513a via the rotation shaft O1. The medical liquid injection device 1B, which uses the wire moving device 50B in the third embodiment as such, also provides an effect similar to that of the first embodiment.

### D. Modification

The disclosed embodiments are not limited to the embodiments described above, and can be performed in a variety of aspects in the range not departing from the spirit, and for example, the following modifications are available.

### Modification 1

The embodiment described above has shown an exemplary configuration of the medical liquid injection devices 1, 1A, and 1B. However, a variety of changes can be employed in the configuration of the medical liquid injection device. For example, the medical liquid injection device may not include a storing part. For example, the medical liquid injection device may include another component not illustrated in the embodiment. Examples of other components to be employed can include a syringe for injecting medical liquid, and a controller for controlling each part in the medical liquid injection device.

### Modification 2

The embodiment described above has shown an exemplary configuration of the catheter 10. However, a variety of changes can be employed in the configuration of the catheter. For example, the catheter may be configured as a multi-lumen catheter including a lumen having another purpose different from that of a lumen for medical liquid. For example, a valve member for regulating back-flow of medical liquid (migration to a wire insertion opening side, a medical liquid supply port side, or the like) may be disposed inside a lumen of the catheter. For example, the catheter may include a mesh member for positioning a needle part in the living body lumen. For example, the catheter may include a balloon member for inhibiting circulation of body fluid flowing in the living body lumen (e.g., blood), and positioning a needle part.

For example, in the axis line O direction, the medical liquid supply port of the connector and the wire insertion opening may be arranged at approximately the same position. For example, in the axis line O direction, the medical liquid supply port of the connector may be disposed closer to the proximal end side than the wire insertion opening. For example, in the axis line O direction, the medical liquid supply port of the connector and the distal end of the wire in maximal winding up of the wire to the storing part are located at approximately the same position. For example, in the axis line O direction, the medical liquid supply port of the connector may be disposed closer to proximal end side than the distal end of the wire in maximal winding-up of the wire to the storing part.

For example, the connector may not include the second extension part, where the medical liquid supply port is formed. In this case, a practitioner may mount a syringe on the wire insertion opening and fill with medical liquid, then remove the syringe, and mount the storing part on the same wire insertion opening and manipulate the wire.

### Modification 3

The embodiment described above has shown an exemplary configuration of the wire moving devices 50 and 50A. However, a variety of changes can be employed in the configuration of the wire moving device. For example, it is possible to freely determine the reference circle diameter L11 of the first gear, the reference circle diameter L31 of the third gear, the outer diameter L12 of the wire winding part, and the outer diameter L32 of the first roller. The ratio of the reference circle diameter L31 of the third gear to the outer diameter L32 of the first roller (L31/L32) may not be less than the ratio of the reference circle diameter L11 of the first gear to the outer diameter L12 of the wire winding part (L11/L12). For example, the first gear and the third gear may not have inner teeth and outer teeth. In this case, the first gear and the wire winding part may be joined with an any bond such as an epoxy-based adhesive. Similarly, the third gear and the first roller may be joined with an any bond such as an epoxy-based adhesive. For example, the wire moving device may transmit a torque of the handle part or the like to the wire winding part, the wire extrusion part, or the like, using a means other than a gear. Examples of the means to be employed other than a gear include a pulley and a belt, magnetic force, and the like.

### Modification 4

The embodiment described above has shown an exemplary configuration of the wires 80 and 80A. However, a variety of changes can be employed in the configuration of the wire. For example, the outer diameter of the wire may not be approximately constant from the distal end to the proximal end. In this case, the wire may have a portion of the distal end side formed with a relatively small diameter, and a portion of the proximal end side formed with a relatively large diameter. For example, the outer diameter of the wire may be less than 80% of the inner diameter of the lumen. For example, the cross-sectional shape of the wire may not be approximately circular.

### Modification 5

The storage 536 in the embodiment described above may store beforehand information on an advance distance d of the wire 80 for administering a dose per administration V. In such configuration including the storage 536, the controller 537 can omit calculation of an advance distance d of the wire 80 in the first calculation process described above. That is, after acquiring an administration start command for the Tth divisional administration, the controller can perform processes of executing the determination process and the second calculation process, which are described above, based on information from the distance detection sensor 52; comparing, in the comparison process described above, an advance distance d of the wire 80 stored in the storage 536 to an advance distance D of the wire 80 calculated in the second calculation process; and upon finding both identical, terminating rotation of the inner rotating part 532 (handle part 512). In such configuration, another distance detection sensor such as the distance detection sensor 52A may be used instead of the distance detection sensor 52.

### Modification 6

The storage 536 in the embodiment described above may store beforehand information that associates the number of rotation of the inner rotating part 532 with an advance distance of the wire 80. For example, an advance distance of the wire 80 to move forward by a single rotation of the inner rotating part 532 may be stored. Such advance distance of the wire 80 can be acquired based on a gear ratio of the inner rotating part 532 to the gear 513, a distance from the rotation shaft O1 of the wire winding part 511 in the wire 80, or the like. Furthermore, the number of rotation of the inner rotating part 532 required for administration of a dose per administration V (hereinafter also referred to as "the number of rotation required") may be stored. In a configuration including the storage 536 as such, and further including a sensor capable of detecting the number of rotation of the inner rotating part 532 (hereinafter also referred to as "rotation rate detection sensor"), the controller 537 can omit calculation of an advance distance d of the wire 80 in the first calculation process; the determination process; the second calculation process; and the comparison process. That is, after acquiring an administration start command for the Tth divisional administration, the controller can perform a process of terminating rotation of the inner rotating part 532 (handle part 512) upon the rotation rate detection sensor detects that the inner rotating part 532 has rotated in the required number of rotation as described above.

Modification 5 and Modification 6 described above can make the operating panel 31 display the number of administration. Moreover, after all of the number of divided administration Tt are executed, the wire retraction process may be executed based on a command input via the operating panel 31 by a practitioner. Furthermore, instead of the wire retraction process, the wire 80 may be retracted by evulsion of the catheter 10 by a practitioner without execution of the wire retraction process, after executing all of the number of divided administration Tt.

The configuration of the medical liquid injection device in the embodiment described above, and the configuration of the medical liquid injection device, the catheter, the wire moving device, the storing part, and the wire in the modification described above may be appropriately combined.

The aspects have been described based on the embodiments and the modifications so far, but the embodiments of the aspect as described above are for the purpose of facilitating understanding the aspects, and do not limit the aspects. While the aspects can be changed or modified without departing from the spirit and scope of the claims, the aspect encompasses the equivalent thereof. In addition, the technical feature thereof can be appropriately deleted unless the technical characteristics is described as essential herein.

### DESCRIPTION OF REFERENCE NUMERALS

1 medical liquid injection device
1A medical liquid injection device
1B medical liquid injection device
10 catheter
10L lumen
11 hollow shaft
11d distal end part
12 needle part
12d distal end part
12p proximal end part
13 connector
31 operating panel
50 wire moving device
50A wire moving device
50B wire moving device
51 wire driver part
51B wire driver part
52 distance detection sensor
52A distance detection sensor
53 CPU built-in case
53B CPU built-in case
60 storing part
61 housing
61o opening
62 valve member
80 wire
80A wire
81 core shaft
85 coating part
85A coating part
86 marker part
86A marker part
131 first extension part
131o wire insertion opening
132 blade part
133 second extension part
133o medical liquid supply port
511 wire winding part
512 handle part
513 gear
513a first gear
513ae outer teeth
513ai inner teeth
513b second gear
513c third gear
513ce outer teeth
513ci inner teeth
514 wire extrusion part
514a first roller
514b second roller
519 coating part
531 outer case
531B outer case
532 inner rotating part
532B inner rotating part
532a cavity part
533 driving control device
534 motor
535 communicator
536 storage
537 controller
D1 first direction
D2 second direction
DS medical liquid
O1 rotation shaft
O axis line

## Claims

1. A medical liquid injection device comprising:
a catheter with a hollow shaft having a lumen formed inside to contain medical liquid,
a wire to be inserted into the lumen, and
a wire moving device to move the wire forward inside the lumen from a proximal end to a distal end side in accordance with a control signal, thereby ejecting a predetermined amount of the medical liquid from the distal end of the hollow shaft.

2. The medical liquid injection device according to claim 1,
wherein the catheter has a hollow needle part at a distal end of the hollow shaft.

3. The medical liquid injection device according to claim 1 or claim 2,
wherein the wire moving device has
a wire driver part to move the wire forward,
a distance detection sensor to detect an advance distance of the wire, and
a control device, and
wherein the control device performs a process of terminating operation of the wire driver part upon determining that the advance distance of the wire derived based on a detection result of the distance detection sensor is a distance corresponding to the predetermined amount.

4. The medical liquid injection device according to claim 3,
wherein the wire moving device has the wire driver part removably configured with the control device.

5. The medical liquid injection device according to claim 3 or claim 4,
wherein the wire driver part moves the wire forward by sending the wire to the lumen and moves the wire backward by winding up the wire from the lumen.

6. The medical liquid injection device according to any one of claim 3 to claim 5,
wherein the wire driver part has
a wire winding part having winding of a portion of a proximal end side of the wire, and
a handle part connected to the wire winding part, and
wherein the wire winding part sends and winds up the wire in a length corresponding to a rotation amount of the handle part.

7. The medical liquid injection device according to claim 6, comprising:
a storing part connected to a proximal end part of the catheter and containing a portion of the proximal end side of the wire and a portion of the wire driver part.

8. The medical liquid injection device according to claim 7,
wherein the wire moving device further comprises a wire extrusion part to extrude, toward the lumen, the wire sent from the wire winding part, and
wherein the wire extrusion part comprises a first roller and a second roller to rotate corresponding to rotation of the handle part, and holds and extrudes the wire by the first roller and the second roller.

9. The medical liquid injection device according to claim 8,
wherein the storing part further comprises a valve member, the valve member being disposed between the wire extrusion part and the proximal end part of the catheter and regulating migration of the medical liquid in the lumen into the storing part.

10. The medical liquid injection device according to any one of claim 6 to claim 9,
wherein the catheter further comprises a connector, the connecter having a medical liquid supply port for supplying the medical liquid to the lumen and a wire insertion opening for inserting the wire into the lumen, the medical liquid supply port being connected to a proximal end part of the hollow shaft and communicating with the lumen, and
wherein the medical liquid supply port is disposed closer to the distal end than a distal end of the wire in maximal winding-up of the wire to the wire winding part by rotation of the handle part.

11. The medical liquid injection device according to claim 10,
wherein the connector further comprises:
a first extension part extending in a longitudinal direction of the catheter, and
a second extension part extending from a lateral face of the first extension part in the vicinity of a distal end of the first extension part in a direction different from that of the first extension part, and
wherein the medical liquid supply port is formed in a distal end part of the second extension part, and the wire insertion opening is formed in a proximal end part of the first extension part.

12. The medical liquid injection device according to any one of claim 1 to claim 11,
wherein an outer diameter of the wire is approximately constant from the distal end to the proximal end.

13. The medical liquid injection device according to any one of claim 1 to claim 12,
wherein each of a cross-sectional shape of the lumen and a cross-sectional shape of the wire is approximately circular, and
wherein an outer diameter of the wire is approximately the same as an inner diameter of the lumen.
